# EUROPEAN PATENT SPECIFICATION

(11) **EP 3 158 914 B1**
(45) Date of publication and mention of the grant of the patent: **25.11.2020**
(21) Application number: 16194804.7
(22) Date of filing: 20.10.2016
(51) Int. Cl.: A61B 1/00, A61B 1/005, B21F 35/00

(54) **PLASTIC DEFORMATION METHOD OF A TIGHTLY WOUND COIL SPRING**
VERFAHREN ZUR PLASTISCHEN VERFORMUNG EINER ENG GEWUNDENEN SPIRALFEDER
PROCÉDÉ DE DÉFORMATION PLASTIQUE DE RESSORT À ENROULEMENT SERRÉ

(30) Priority: 21.10.2015 JP 2015207079
(43) Date of publication of application: 26.04.2017
(73) Proprietor: Fujifilm Corporation, Minato-ku Tokyo 106-8620 (JP)
(72) Inventor: ASAOKA, Takuro, Kanagawa, 258-8538 (JP); UEDA, Yoshihiro, Kanagawa, 258-8538 (JP)
(74) Representative: Klunker IP Patentanwälte PartG mbB

(56) References cited:
- JP-A- 2006 122 962
- JP-A- 2009 061 173
- JP-A- 2012 122 280
- US-A1- 2015 087 905
- US-B1- 8 069 881

## Description

### BACKGROUND OF THE INVENTION

### Field of the Invention

The present invention relates to a plastic deformation method of a tightly wound coil spring and particularly to a plastic deformation method of a tightly wound coil spring for improving durability of a tightly wound coil spring disposed inside an endoscope insertion portion.

### Description of the Related Art

JP 2009 061173 A teaches an endoscope apparatus comprising an insertion section capable of being bent and inserted into the inside of a subject.

JP 2006 122962 A teaches a spiral spring manufacturing method in which inter-spring via space can be freely set.

US 2015/087905 A1 teaches an endoscope, a flexible device of an elongated tube having a variable stiffness device.

US 8 069 881 B1 discloses a method in which a coil spring is radially expanded. To this end, an expansion force is used such that an outer diameter portion of the coil spring is expanded radially from an initial size to an expanded size.

Conventionally, medical diagnosis using an endoscope has been widely carried out in the medical field. Particularly an image pickup element such as a CCD (Charge Coupled Device) is incorporated in a tip end portion of an endoscope insertion portion (hereinafter referred to as an insertion portion) to be inserted into a body cavity so as to pick up an image of an inside of the body cavity, signal processing is executed in a processor device and displayed on a monitor, and this is observed by a medical doctor and used for diagnosis or a treatment tool is inserted through a channel for inserting a treatment tool, and treatment such as sampling and excision of a polyp is performed, for example.

The endoscope is roughly constituted in general by an operation portion gripped and operated by an operator and the insertion portion to be inserted into the body cavity or the like provided consecutively to this operation portion, and a universal cable to be connected to a connector portion or the like is withdrawn from the operation portion. Then, the universal cable is extended from the operation portion and has the other end portion detachably connected to a light source device or a processor device.

The insertion portion of the endoscope has a soft portion having flexibility so that it can be inserted into an insertion path which is curved complicatedly. However, there is a problem that due to this flexibility, a direction of a tip end side of the insertion portion is not determined and the insertion portion cannot be inserted easily in a target direction. Moreover, when it is inserted into the body cavity, it is desirable that the insertion portion is fixed in a shape at that time for performing some treatment or observation in some cases.

Thus, an endoscope having a hardness adjusting device constituted by a coil and a wire inserted into this coil disposed inside the soft portion is disclosed in Japanese Unexamined Patent Application Publication No. 2001-258828. According to the endoscope of Japanese Unexamined Patent Application Publication No. 2001-258828, by rotationally moving and operating a hardness adjustment knob provided on the operation portion by the operator for pulling the wire and compressing the coil in an axial direction, hardness of the soft portion can be increased. That is, flexibility of the soft portion can be controlled by the hardness adjusting device.

As disclosed in Japanese Unexamined Patent Application Publication No. 2001-258828, in the endoscope having the hardness adjusting device, if the hardness adjusting device is repeatedly used in many inspections, the coil is gradually contracted by plastic deformation, and if the hardness is set/adjusted to the maximum, a hardness change amount becomes small, and desired functions are lost, which is a problem.

Thus, in a hardness adjusting apparatus of Japanese Unexamined Patent Application Publication No. 2001-258828, in order to solve the aforementioned problem, a predetermined amount of a compression force is applied to the coil so that a length dimension of the coil is deformed to a short length dimension different from a natural dimension and is disposed inside the soft portion.

Specifically, before the wire and the coil are assembled to the insertion portion, tip end sides of the wire and the coil are fixed by brazing. Moreover, a coil stopper is fixed to a base end side of the coil. Then, by holding the coil stopper and by pulling/operating the wire once or several times with a predetermined force amount so as to apply the compression force to the coil so that the length dimension of the coil is changed to a length shorter than the natural length. Subsequently, in this state, the wire stopper is fixed to the wire by brazing. After that, the wire and the coil are assembled to insides of the insertion portion and the operation portion.

According to Japanese Unexamined Patent Application Publication No. 2001-258828, a predetermined amount (predetermined force amount, predetermined number of times, predetermined time) of the compression force is applied to the coil in advance, and the coil is plastically deformed by applying the compression force in advance in initial deformation, that is, in a stage before a user starts using and thus, if the user repeatedly uses a hardness adjusting function, deterioration of this function is made as small as possible.

### SUMMARY OF THE INVENTION

However, since the hardness adjusting apparatus of Japanese Unexamined Patent Application Publication No. 2001-258828 applies the compression force to the coil by pulling the wire in a state where the coil and the wire are fixed on the tip end sides, a compression force exceeding strength of the wire cannot be applied to the coil.

Moreover, if the compression force applied to the coil is increased, the coil begins meandering deformation by the compression force and thus, a high compression force cannot be efficiently applied to the coil, and improvement of durability of the coil is limited.

Furthermore, since the base end side of the wire is fixed by a chuck and the wire is pulled in actuality, at least a portion of the wire fixed by the chuck may be damaged. Thus, a lengthy wire is inserted into the coil in advance, and the base end side of the wire is fixed by the chuck and the compression force is applied to the coil and then, a work of cutting off the base end side of the wire damaged by the chuck needs to be performed. Thus, there is a problem that facility becomes bulky and workability is poor.

Moreover, there is a similar problem of improvement of durability of the coil also for a tightly wound coil spring for curving operation wire guide for guiding a wire for curving operation of a curved portion of the endoscope insertion portion.

The present invention was made in view of such circumstances and has an object to provide a plastic deformation method of a tightly wound coil spring which can improve durability of the tightly wound coil spring with simple facility and with good workability.

In order to achieve the aforementioned object, a plastic deformation method of a tightly wound coil spring according to the present invention includes the steps of claim 1. Since the compression force in the tightly wound coil spring axial direction is applied to the tightly wound coil spring in the state where the deformation of the tightly wound coil spring in the tightly wound coil spring radial direction is regulated, the tightly wound coil spring is given the compression force in a state where meandering deformation is regulated. As a result, a high compression force can be efficiently applied to the tightly wound coil spring. Thus, durability of the tightly wound coil spring can be improved with good workability and with the simple facility which only regulates the deformation in the tightly wound coil spring radial direction.

In the regulating process, the tightly wound coil spring is inserted into a cylindrical body, and the deformation of the tightly wound coil spring in the tightly wound coil spring radial direction is regulated by the cylindrical body.

According to one aspect of the present invention, since the deformation of the tightly wound coil spring in the tightly wound coil spring radial direction can be regulated only by inserting the tightly wound coil spring into the cylindrical body, the regulating process can be performed with simpler facility.

In one aspect of the present invention, it is preferable that a length of the cylindrical body in a cylindrical body axial direction is configured to be longer than a length of the tightly wound coil spring in the tightly wound coil spring axial direction before the compression force is applied, and in the plastic deformation process, the compression force is applied to the tightly wound coil spring inserted into the cylindrical body from one end or from both ends in the tightly wound coil spring axial direction.

According to the one aspect of the present invention, since a whole length of the tightly wound coil spring can be inserted into the cylindrical body, the compression force can be applied to the tightly wound coil spring in a state where meandering deformation is prevented in the whole length of the tightly wound coil spring.

In one aspect of the present invention, it is preferable that, in the plastic deformation process, a process of applying the compression force in the tightly wound coil spring axial direction to the tightly wound coil spring and a process of releasing the compression force to the tightly wound coil spring are repeated at least twice or more.

According to the one aspect of the present invention, by repeating the process of applying the compression force and the process of releasing the compression force, the tightly wound coil spring can be plastically deformed efficiently.

In one aspect of the present invention, it is preferable that the tightly wound coil spring is a tightly wound coil spring for hardness adjustment which adjusts hardness of an endoscope soft portion.

In one aspect of the present invention, it is preferable that the tightly wound coil spring is a tightly wound coil spring constituted such that a wire is inserted into the tightly wound coil spring, a tip end portion of the tightly wound coil spring is connected to a tip end portion of the wire directly or indirectly and is compressed in a coil spring axial direction by a change in a relative distance in a longitudinal axis direction of the endoscope insertion portion between a base end portion of the tightly wound coil spring and a base end portion of the wire.

In one aspect of the present invention, it is preferable that the compression force is at least five or more times a maximum compression force in the tightly wound coil spring axial direction applied to the tightly wound coil spring when the endoscope soft portion is in a linear state and the hardness of the endoscope soft portion is maximized.

According to the one aspect of the present invention, the tightly wound coil spring can be plastically deformed by a deformation amount by which durability of the tightly wound coil spring can be sufficiently obtained.

In one aspect of the present invention, it is preferable that the tightly wound coil spring is a tightly wound coil spring for curving operation wire guide which guides a wire performing a curving operation of the endoscope curved portion.

In one aspect of the present invention, it is preferable that the tightly wound coil spring is a tightly wound coil spring into which a wire for the curving operation of a curved portion of the endoscope insertion portion is inserted.

According to the plastic deformation method of the tightly wound coil spring of the present invention, durability of the tightly wound coil spring can be improved with simple facility and good workability.

### BRIEF DESCRIPTION OF THE DRAWINGS

Fig. 1 is a sectional view of an endoscope;
Fig. 2 is a flowchart of a plastic deformation method of an embodiment;
Figs. 3A, 3B, and 3C are explanatory views over time of the plastic deformation method of the embodiment;
Fig. 4A is a sectional view of an essential part of a tightly wound coil spring before plastic deformation and Fig. 4B is a sectional view of an essential part of the tightly wound coil spring after the plastic deformation; and
Fig. 5 is a sectional view of an endoscope insertion portion showing the tightly wound coil spring which guides a curving operation wire.

### DETAILED DESCRIPTION OF THE EMBODIMENTS

A plastic deformation method of a tightly wound coil spring according to the present invention will be described below by referring to the attached drawings.

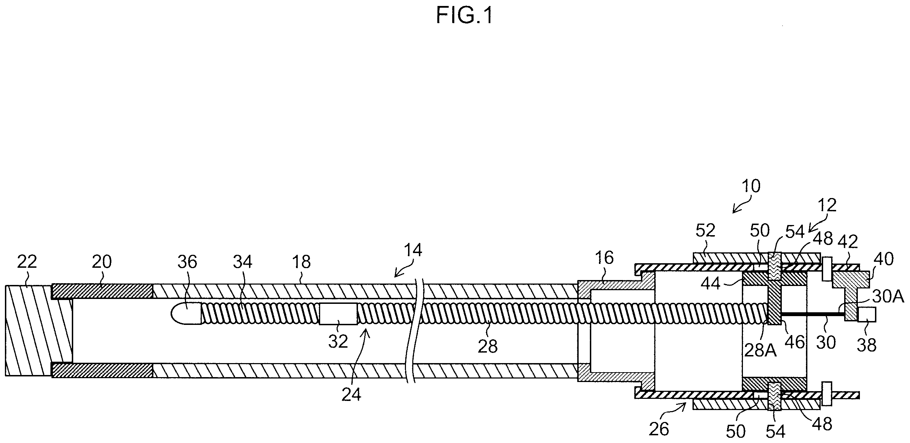

Fig. 1 is a sectional view of an endoscope 10 having a tightly wound coil spring 28 plastically deformed by a plastic deformation method of an embodiment.

### [Constitution of endoscope 10]

The endoscope 10 of the embodiment includes an operation portion 12 and an insertion portion 14 which is an endoscope insertion portion provided consecutively to the operation portion 12.

A base end side of the insertion portion 14 is provided consecutively to a tip end side of the operation portion 12 through a cover member 16 which is a bending stopper. The insertion portion 14 includes a soft portion 18 which is an endoscope soft portion, a curved portion 20 which is an endoscope curved portion, and a tip-end hard portion 22 from the base end portion to the tip end portion of the insertion portion 14.

In the endoscope 10, a hardness adjusting apparatus is provided. The hardness adjusting apparatus includes a hardness adjusting device 24 which adjusts hardness of the soft portion 18 and a hardness adjusting portion 26 which operates the hardness adjusting device 24.

### <Hardness adjusting device 24, hardness adjusting portion 26>

In Fig. 1, the insertion portion 14 is illustrated in a broken way and in order to avoid complicatedness of the drawing, the hardness adjusting device 24 including a tightly wound coil spring 28 and a wire 30 which are tightly wound coil spring for hardness adjustment is mainly illustrated.

As a method of the hardness adjusting portion 26 which changes a compressed state of the tightly wound coil spring 28, there are two methods, that is, a wire pulling method of pulling a base end portion 30A of the wire 30 to a base end side with respect to a base end portion 28A of the tightly wound coil spring 28 and a coil compression method of pushing in the base end portion 28A of the tightly wound coil spring 28 toward a tip end portion side. The tightly wound coil spring 28 used in the both methods has the wire 30 inserted inside, and the tip end portion of the tightly wound coil spring 28 is connected to a tip end portion of the wire 30 directly or indirectly as will be described later, and it is a tightly wound coil spring compressed in a coil spring axial direction by means of a change in a relative distance in a longitudinal axis direction of the insertion portion 14 between the base end portion 28A of the tightly wound coil spring 28 and a base end portion 30A of the wire 30. In the embodiment below, explanation will be made in the coil compression method.

The tightly wound coil spring 28 is provided from the soft portion 18 to the operation portion 12. Moreover, the wire 30 is provided from the soft portion 18 to the operation portion 12 and is inserted into a hollow portion of the tightly wound coil spring 28. Each of the tip end portions of the wire 30 and the tightly wound coil spring 28 is fixed to a base end portion of a relay fixture 32 by a fixing method such as brazing.

To a tip end portion of the relay fixture 32, the base end portion of the tightly wound coil spring 34 which has a short length and is a cushion member is connected, and a fixture 36 is fixed to the tip end portion of the tightly wound coil spring 34. The relay fixture 32, the tightly wound coil spring 34, and the fixture 36 are not fixed to the insertion portion 14 and thus, a tip end portion of the hardness adjusting device 24 is constituted as a free end.

On the other hand, the base end portion 30A of the wire 30 is extended to a base end side from the base end portion 28A of the tightly wound coil spring 28 and is inserted into a wire sleeve 38 and fixed. The wire sleeve 38 is fixed to a frame 42 of the operation portion 12 through a wire fixing block 40 provided on the operation portion 12 and is constituted as a fixed end.

The base end portion 28A of the tightly wound coil spring 28 is supported in contact by a compression portion 46 provided on a push ring 44. The compression portion 46 applies a compression force in a rightly wound coil spring axial direction to the tightly wound coil spring 28 by advancing the push ring 44 to the tip end side. That is, by advancing the push ring 44 to the tip end side, the tightly wound coil spring 28 is compressed in a tightly wound coil spring axial direction. Then, by retreating the push ring 44 to the base end side, the tightly wound coil spring 28 is returned to a natural length from the compressed state.

On an outer circumferential surface of the push ring 44, at least two cam pins 48 are projected. The cam pin 48 is engaged with a cam groove 54 in a cam ring 52 externally mounted on the frame 42 through a linear groove 50 provided in the frame 42. Therefore, when the cam ring 52 is rotationally moved, the push ring 44 can move forward/backward to the tip end side or to the base end side through the cam pins 48 guided by the linear grooves 50 and the cam grooves 54.

Outline constitution of the endoscope 10 and an action of the hardness adjusting apparatus have been described above. Subsequently, a plastic deformation method of the tightly wound coil spring 28 for hardness adjustment which adjusts the hardness of the soft portion 18 of the endoscope 10 will be described.

### [Plastic deformation method of tightly wound coil spring 28]

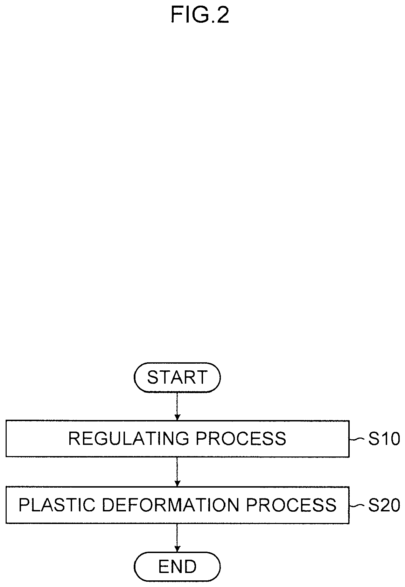

Fig. 2 is a flowchart of the plastic deformation method of the embodiment.

The plastic deformation method of the embodiment includes a regulating process (Step) S10 which regulates deformation of the tightly wound coil spring 28 in a tightly wound coil spring radial direction and a plastic deformation process S20 which plastically deforms the tightly wound coil spring 28 by applying the compression force in the tightly wound coil spring axial direction to the tightly wound coil spring 28 in a state where deformation of the tightly wound coil spring 28 in the tightly wound coil spring radial direction is regulated.

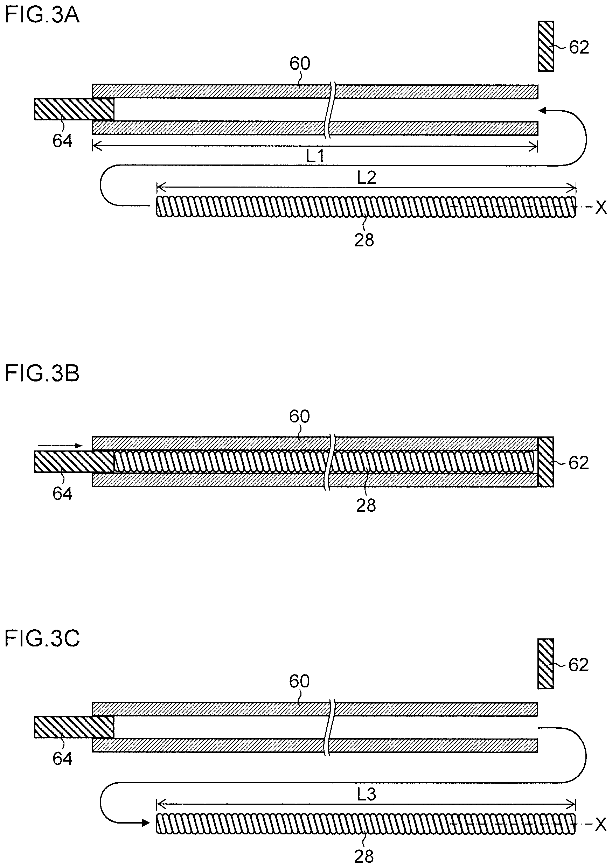

Figs. 3A, 3B, and 3C are explanatory views illustrating an example of the plastic deformation method of the embodiment over time.

In the regulating process S10 (see Fig. 2), as illustrated in the sectional views in Figs. 3A and 3B, the tightly wound coil spring 28 is inserted into a pipe 60 made of metal, which is a cylindrical body, so as to regulate deformation of the tightly wound coil spring 28 in the tightly wound coil spring radial direction by an inner circumferential surface of the pipe 60. That is, only by inserting the tightly wound coil spring 28 into the pipe 60, deformation of the tightly wound coil spring 28 in the tightly wound coil spring radial direction can be regulated. Thus, the regulating process S10 can be performed with simpler facility.

The member is not limited to the pipe 60 as long as it can regulate the deformation of the tightly wound coil spring 28 in the tightly wound coil spring radial direction. For example, the deformation of the tightly wound coil spring 28 in the tightly wound coil spring radial direction may be regulated by dividing a die member including an insertion hole of the tightly wound coil spring 28 into two parts in the tightly wound coil spring axial direction and by using a semicircular groove which becomes an insertion hole, provided in each of facing surfaces of the die members. Moreover, the pipe 60 is not limited to a cylindrical shape but may be a rectangular or square tube.

Moreover, the pipe 60 is configured to have a length L1 in the cylindrical body axial direction longer than a length L2 of the tightly wound coil spring 28 in the tightly wound coil spring axial direction (X-direction in Figs. 3A and 3B) before the compression force is applied.

Then, in the plastic deformation process S20 (see Fig. 2) subsequent to the regulating process S10, the compression force is applied from one end or both ends in the tightly wound coil spring axial direction to the tightly wound coil spring 28 inserted into the pipe 60. As illustrated in Fig. 3B, the tightly wound coil spring 28 is inserted into the pipe 60 and a right-end opening portion of the pipe 60 whose both ends are open is closed by a cap 62. Then, a rod 64 is inserted into the pipe 60 from a left-end opening portion of the pipe 60, and the compression force is applied to the tightly wound coil spring 28 by a pressing force indicated by an arrow of the rod 64. Since an outer diameter of the rod 64 is substantially equal to an outer diameter of the tightly wound coil spring 28, the tightly wound coil spring 28 can be favorably compressed in the tightly wound coil spring axial direction. The compression force may be applied to the tightly wound coil spring 28 by inserting two rods 64 from the opening portions on the both ends of the pipe 60.

According to the plastic deformation process S20 in Fig. 3B, since a whole length of the tightly wound coil spring 28 can be inserted into the pipe 60, the compression force can be applied to the tightly wound coil spring 28 in a state where meandering deformation is prevented in the whole length of the tightly wound coil spring 28.

After that, as illustrated in Fig. 3C, the cap 62 is removed from the pipe 60, and the tightly wound coil spring 28 is taken out of the pipe 60. The plastic deformation process S20 is finished as above.

As described above, the plastic deformation method of the embodiment applies the compression force in the tightly wound coil spring axial direction to the tightly wound coil spring 28 in the state where the deformation of the tightly wound coil spring 28 in the tightly wound coil spring radial direction is regulated with respect to the single body tightly wound coil spring 28. As a result, since the compression force is applied to the tightly wound coil spring 28 in the state where the meandering deformation is regulated, a high compression force can be efficiently applied to the tightly wound coil spring 28.

Thus, according to the plastic deformation method of the embodiment, durability of the tightly wound coil spring 28 can be improved with good workability with simple facility which only regulates the deformation in the tightly wound coil spring radial direction.

Moreover, it is preferable that, in the plastic deformation process S20, the compression force applied from the rod 64 to the tightly wound coil spring 28 is at least five or more times a maximum compression force in the tightly wound coil spring axial direction applied to the tightly wound coil spring 28, that is, a compression force of 1000 N or more, for example, when the soft portion 18 in Fig. 1 is brought into a linear state and hardness of the soft portion 18 is maximized by the hardness adjusting apparatus. As a result, the tightly wound coil spring 28 can be plastically deformed effectively to a deformation amount with which durability of the tightly wound coil spring 28 can be sufficiently obtained.

On the other hand, in the plastic deformation method of Japanese Unexamined Patent Application Publication No. 2001-258828 or the like in which the compression force is applied to the tightly wound coil spring by pulling the wire, if the compression force at 1000 N or more is applied, the wire is disconnected due to the size of the compression force in some cases. Thus, since it is difficult for the plastic deformation method of Japanese Unexamined Patent Application Publication No. 2001-258828 or the like to apply the compression force at 1000 N or more to the tightly wound coil spring, the tightly wound coil spring cannot be plastically deformed effectively.

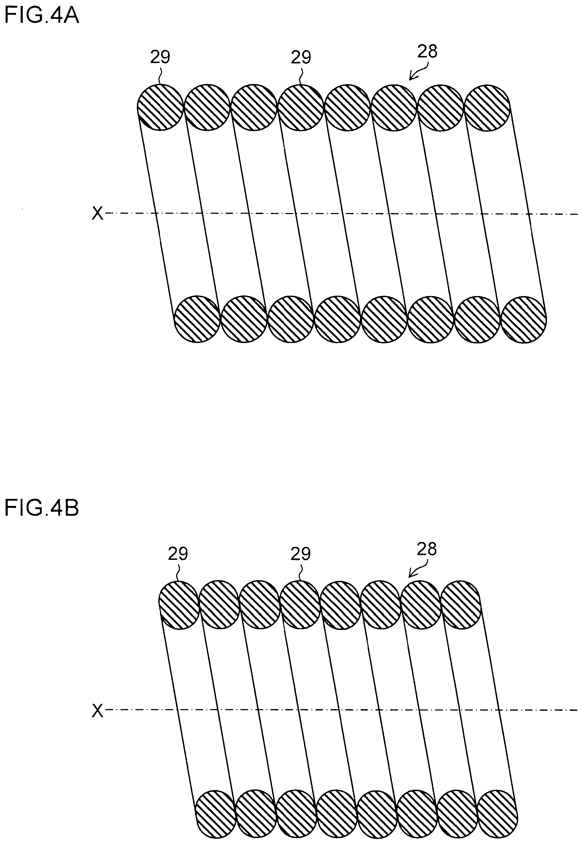

On the other hand, according to the plastic deformation method of the embodiment, the tightly wound coil spring 28 before the plastic deformation illustrated in the sectional view of the essential part in Fig. 4A can be plastically deformed effectively as shown in the tightly wound coil spring 28 after the plastic deformation illustrated in the sectional view of the essential part in Fig. 4B.

The tightly wound coil spring 28 before the plastic deformation in Fig. 4A has its element wire 29 with a circular section in contact with the adjacent element wire 29. On the other hand, the tightly wound coil spring 28 after the plastic deformation in Fig. 4B is plastically deformed to a shape in which a part of the circular shape of its element wire 29 is crushed by the applied large compression force, and the adjacent element wires 29 are pressed into contact with each other. The sectional shape of the tightly wound coil spring 28 in Fig. 4B is an oval but it is not limited to an oval but can be plastically deformed to a flat circular shape or a long circular shape in some cases. Thus, a length L3 of the tightly wound coil spring 28 in the tightly wound coil spring axial direction after the plastic deformation illustrated in Fig. 3C is shorter than the length L2 before the plastic deformation by a portion of plastic deformation of the element wire 29 from a circular shape to an oval shape, and favorable plastic deformation is achieved.

Moreover, it is preferable that the plastic deformation process S20 repeats the process of applying the compression force in the tightly wound coil spring axial direction to the tightly wound coil spring 28 and the process of releasing the compression force to the tightly wound coil spring 28 at least twice or more. As a result, since the compression force can be efficiently applied to the tightly wound coil spring 28, the tightly wound coil spring 28 can be plastically deformed efficiently. In this case, it is preferable that the compression force is also 1000 N or more.

In the aforementioned embodiment, the plastic deformation method of the tightly wound coil spring 28 in which hardness of the endoscope soft portion is adjusted is described, but the plastic deformation method of the present invention can be also applied to a plastic deformation method of a tightly wound coil spring for curving operation wire guide which guides a wire for curving operation of the endoscope curved portion.

Here, the tightly wound coil spring for curving operation wire guide will be described in brief.

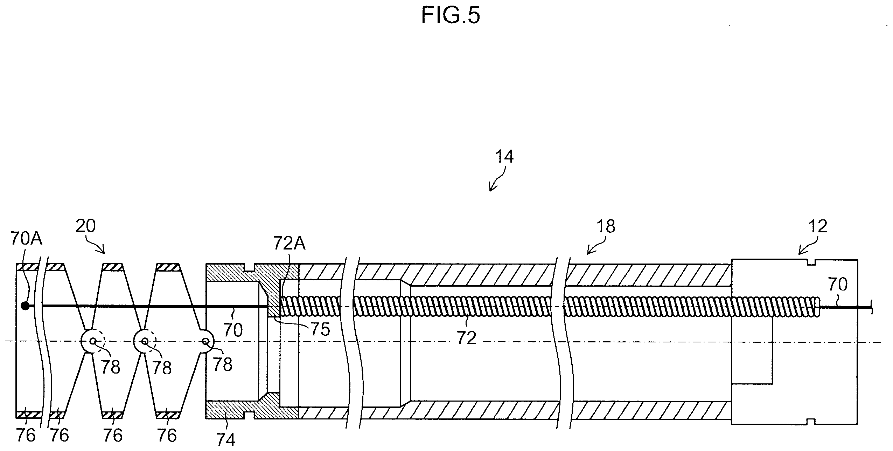

Fig. 5 is a sectional view of the insertion portion 14 illustrating a tightly wound coil spring for curving operation wire guide (hereinafter referred to as a tightly wound coil spring) 72 which guides a curving operation wire 70.

A curved portion 20 includes a base 74 connected to a tip end side of the soft portion 18 and a plurality of angle rings 76 provided consecutively to the base 74. The base 74 and the angle rings 76 are consecutively connected rotatably through a pin 78, respectively, and are curved/operated by a pulling operation of the curving operation wire 70. A tightly wound coil spring 72 guides the curving operation wire 70 by being arranged on the tip end side of the soft portion 18 from the operation portion 12 and having the curving operation wire 70 inserted therein.

The aforementioned plastic deformation method of the present invention is applied to the tightly wound coil spring 72 constituted as above. That is, with respect to the single body tightly wound coil spring 72, the compression force in the tightly wound coil spring axial direction is applied to the tightly wound coil spring 72 in a state where deformation of the tightly wound coil spring 72 in the tightly wound coil spring radial direction is regulated. As a result, since the compression force is applied to the tightly wound coil spring 72 in the state where meandering deformation is regulated, a high compression force can be efficiently applied to the tightly wound coil spring 72.

Thus, according to the plastic deformation method of the embodiment, durability of the tightly wound coil spring 72 can be improved with good workability and with simple facility which only regulates the deformation in the tightly wound coil spring radial direction.

A tip end portion 72A of the tightly wound coil spring 72 is fixed to a flange portion 75 of the base 74, and a tip end portion 70A of the curving operation wire 70 is fixed to the angle ring 76 arranged on the tip end side of the curved portion 20. That is, the tip end portion 72A of the tightly wound coil spring 72 is indirectly connected to the tip end portion 70A of the curving operation wire 70 through the base 74 and the plurality of angle rings 76. Moreover, to the tightly wound coil spring 72, the compression force is applied by curving operation of the curved portion 20 by a pulling operation of the curving operation wire 70 to the base end side.

On the other hand, the tightly wound coil spring 28 for hardness adjustment and the wire 30 illustrated Fig. 1 have their respective tip end portions directly fixed to the base end portion of the relay fixture 32 by a fixing method such as brazing. Moreover, to the tightly wound coil spring 28, the compression force in the tightly wound coil spring axial direction is applied by advancing the push ring 44 to the tip end side.

That is, the tightly wound coil spring 28 for hardness adjustment illustrated in Fig. 1 and the tightly wound coil spring 72 for curving operation illustrated in Fig. 5 are common in the structure in which the wire is inserted into the tightly wound coil spring and the tip end portion of the tightly wound coil spring is connected to the tip end portion of the wire directly or indirectly and in the action of applying the compression force to the coil.

Thus, the plastic deformation method of the embodiment can be applied to the tightly wound coil spring 72 for curving operation having the common structure and action with the tightly wound coil spring 28 for hardness adjustment, and durability of the tightly wound coil spring 72 can be improved with good workability with simple facility similarly to the tightly wound coil spring 28.

## Claims

1. A plastic deformation method of a tightly wound coil spring (28) for improving durability of the tightly wound coil spring (28), comprising:
a regulating step (S10) of regulating deformation of the tightly wound coil spring (28) in a coil spring radial direction by a cylindrical body (60) adapted to prevent the tightly wound coil spring (28) from being meanderingly deformed by inserting the tightly wound coil spring (28) into the cylindrical body (60), before the tightly wound coil spring (28) is fixed to an endoscope insertion portion (14); and
a plastic deformation step (S20) of plastically deforming the tightly wound coil spring (28) by applying a compression force in a coil spring axial direction to the tightly wound coil spring (28) in a state where meandering deformation of the tightly wound coil spring (28) in the coil spring radial direction is regulated.

2. The plastic deformation method of a tightly wound coil spring (28) according to claim 1, wherein
a length of the cylindrical body (60) in a cylindrical body axial direction is configured to be longer than a length of the tightly wound coil spring (28) in the coil spring axial direction before the compression force is applied; and
in the plastic deformation step (S20), the compression force is applied to the tightly wound coil spring (28) inserted into the cylindrical body (60) from one end or from both ends in the coil spring axial direction.

3. The plastic deformation method of a tightly wound coil spring (28) according to any one of claims 1 or 2, wherein
in the plastic deformation step (S20), a step of applying the compression force in the coil spring axial direction to the tightly wound coil spring (28) and a step of releasing the compression force to the tightly wound coil spring (28) are repeated at least twice or more.

4. The plastic deformation method of a tightly wound coil spring (28) according to any one of claims 1 to 3, wherein
the tightly wound coil spring (28) is a tightly wound coil spring (28) for hardness adjustment which adjusts hardness of an endoscope soft portion (18).

5. The plastic deformation method of a tightly wound coil spring (28) according to claim 4, wherein
a wire is inserted into the tightly wound coil spring (28), a tip end portion of the tightly wound coil spring (28) is connected to a tip end portion of the wire directly or indirectly and is compressed in a coil spring axial direction by a change in a relative distance in a longitudinal axis direction of the endoscope insertion portion between a base end portion of the tightly wound coil spring (28) and a base end portion of the wire.

6. The plastic deformation method of a tightly wound coil spring (28) according to any one of claims 1 to 5, wherein
the compression force is at least five or more times a maximum compression force in the coil spring axial direction applied to the tightly wound coil spring (28) when an endoscope soft portion (18) is in a linear state and the hardness of the endoscope soft portion is maximized.

7. The plastic deformation method of a tightly wound coil spring (28) according to any one of claims 1 to 3, wherein
the tightly wound coil spring (28) is a tightly wound coil spring (28) for a curving operation wire guide which guides a wire (70) performing a curving operation of an endoscope curved portion (20).

8. The plastic deformation method of a tightly wound coil spring (28) according to claim 7, wherein
the tightly wound coil spring (28) is a tightly wound coil spring (28) into which a wire for curving operation of a curved portion of the endoscope insertion portion is inserted.

## Patentansprüche

1. Verfahren zum plastischen Verformen einer eng gewickelten Spiralfeder (28) zum Verbessern der Haltbarkeit der eng gewickelten Spiralfeder (28), umfassend:
einen Einstellschritt (S10) zum Einstellen einer Verformung der eng gewickelten Spiralfeder (28) in einer radialen Richtung der Spiralfeder mittels eines zylindrischen Körpers (60), um zu verhindern, dass sich die eng gewickelte Spiralfeder (28) mäandrierend verformt, wozu die eng gewickelte Spiralfeder (28) in den zylindrischen Körper (60) eingeführt wird, bevor die eng gewickelte Spiralfeder (28) an einem Endoskop-Einführteil (14) fixiert wird; und
einen Schritt (S20) des plastischen Verformens der eng gewickelten Spiralfeder (28) durch Aufbringen einer Druckkraft in axialer Richtung der Spiralfeder auf die eng gewickelte Spiralfeder (28) in einem Zustand, in welchem eine mäandrierende Verformung der eng gewickelten Spiralfeder (28) in deren radialer Richtung gesteuert wird.

2. Verfahren zum plastischen Verformen einer eng gewickelten Spiralfeder (28) nach Anspruch 1, bei dem
eine Länge des zylindrischen Körpers (60) in dessen axialer Richtung, die so konfiguriert ist, dass sie länger ist als eine Länge der eng gewickelten Spiralfeder (28) in deren axialer Richtung, bevor die Druckkraft aufgebracht wird; und
in dem Schritt (S20) des plastischen Verformens die Druckkraft auf die eng gewickelte Spiralfeder (28), die in den zylindrischen Körper (60) eingeführt ist, von einem Ende oder von beiden Enden in axialer Richtung der Spiralfeder aufgebracht wird.

3. Verfahren zum plastischen Verformen einer eng gewickelten Spiralfeder (28) nach einem der Ansprüche 1 und 2, bei dem
in dem Schritt (S20) des plastischen Verformens ein Schritt des Aufbringens der Druckkraft in axialer Richtung der Spiralfeder auf die eng gewickelte Spiralfeder (28) und ein Schritt des Nachlassens der Druckkraft auf die eng gewickelte Spiralfeder (28) mindestens zweimal oder mehr wiederholt werden.

4. Verfahren zum plastischen Verformen einer eng gewickelten Spiralfeder (28) nach einem der Ansprüche 1 bis 3, bei dem
die eng gewickelte Spiralfeder (28) eine eng gewickelte Spiralfeder (28) für eine Härteeinstellung ist, die die Härte eines Endoskop-Weichabschnitts (18) einstellt.

5. Verfahren zum plastischen Verformen einer eng gewickelten Spiralfeder (28) nach Anspruch 4, bei dem
in die eng gewickelte Spiralfeder (28) ein Draht eingeführt wird, ein Endbereich der eng gewickelten Spiralfeder (28) mit einem Endbereich des Drahts direkt oder indirekt verbunden wird, und in axialer Richtung der Spiralfeder zusammengedrückt wird durch eine Änderung einer relativen Distanz in Linksachsenrichtung des Endoskop-Einführteils zwischen einem Basisendbereich der eng gewickelten Spiralfeder (28) und einem Basisendbereich des Drahts.

6. Verfahren zum plastischen Verformen einer eng gewickelten Spiralfeder (28) nach einem der Ansprüche 1 bis 5, bei dem
die Druckkraft mindestens 5 mal oder häufiger eine maximale Druckkraft in axialer Richtung der Spiralfeder ist, aufgebracht auf die eng gewickelte Spiralfeder (28), wenn ein Endoskop-Weichbereich (18) sich in einem linearen Zustand befindet und die Härte des Endoskop-Weichbereichs maximiert ist.

7. Verfahren zum plastischen Verformen einer eng gewickelten Spiralfeder (28) nach einem der Ansprüche 1 bis 3, bei dem
die eng gewickelte Spiralfeder (28) eine eng gewickelte Spiralfeder (28) für eine Kurvenbereichs-Drahtführung ist, die einen Draht (70) führt, welcher einen Kurvendurchgang eines gekrümmten Endoskopbereichs (20) ausführt.

8. Verfahren zum plastischen Verformen einer eng gewickelten Spiralfeder (28) nach Anspruch 7, bei dem
die eng gewickelte Spiralfeder (28) eine eng gewickelte Spiralfeder (28) ist, in die ein Draht für einen Kurvendurchgang eines gekrümmten Bereichs des Endoskop-Einführabschnitts eingeführt ist.

## Revendications

1. Procédé de déformation plastique d'un ressort à enroulement serré (28) destiné à améliorer la durabilité du ressort à enroulement serré (28), comprenant :
une étape de régulation (S10) pour réguler la déformation du ressort à enroulement serré (28) dans une direction radiale de ressort à enroulement par un corps cylindrique (60) apte à empêcher que le ressort à enroulement serré (28) ne se déforme en méandres en introduisant le ressort à enroulement serré (28) dans le corps cylindrique (60), avant que le ressort à enroulement serré (28) ne soit fixé sur la portion d'introduction d'endoscope (14), et
une étape de déformation plastique (S20) pour déformer sur le plan plastique le ressort à enroulement serré (28) en appliquant une force de compression dans une direction axiale de ressort à enroulement au ressort à enroulement serré (28) dans un état où la déformation en méandres du ressort à enroulement serré (28) dans la direction radiale de ressort à enroulement est régulée.

2. Procédé de déformation plastique d'un ressort à enroulement serré (28) selon la revendication 1, dans lequel
une longueur du corps cylindrique (60) dans une direction axiale de corps cylindrique est configurée pour être plus longue que la longueur du ressort à enroulement serré (28) dans la direction axiale de ressort à enroulement avant que la force de compression ne soit appliquée, et
lors de l'étape de déformation plastique (S20), la force de compression est appliquée au ressort à enroulement serré (28) introduit dans le corps cylindrique (60) à partir d'une seule extrémité ou des deux extrémités dans la direction axiale de ressort à enroulement.

3. Procédé de déformation plastique d'un ressort à enroulement serré (28) selon la revendication 1 ou 2, dans lequel
lors de l'étape de déformation plastique (S20), une étape d'application de la force de compression dans la direction axiale de ressort à enroulement au ressort à enroulement serré (28) et une étape de relâchement de la force de compression sur le ressort à enroulement serré (28) sont répétées au moins deux fois, ou davantage.

4. Procédé de déformation plastique d'un ressort à enroulement serré (28) selon l'une quelconque des revendications 1 à 3, dans lequel
le ressort à enroulement serré (28) est un ressort à enroulement serré (28) destiné à au réglage de dureté, lequel règle la dureté d'une partie souple d'endoscope (18).

5. Procédé de déformation plastique d'un ressort à enroulement serré (28) selon la revendication 4, dans lequel
un fil est introduit dans le ressort à enroulement serré (28), une portion d'extrémité de pointe du ressort à enroulement serré (28) est raccordée à une portion d'extrémité de pointe du fil directement ou indirectement, et est comprimée dans une direction axiale de ressort à enroulement par une modification d'une distance relative dans une direction d'axe longitudinal de la portion d'introduction d'endoscope entre une portion d'extrémité de base du ressort à enroulement serré (28) et une portion d'extrémité de base du fil.

6. Procédé de déformation plastique d'un ressort à enroulement serré (28) selon l'une quelconque des revendications 1 à 5, dans lequel
la force de compression est égale à au moins cinq fois, ou davantage, la force de compression maximale dans la direction axiale de ressort à enroulement appliquée au ressort à enroulement serré (28) lorsqu'une portion souple d'endoscope (18) se trouve dans un état linéaire et que la dureté de la portion souple d'endoscope est maximisée.

7. Procédé de déformation plastique d'un ressort à enroulement serré (28) selon l'une quelconque des revendications 1 à 3, dans lequel
le ressort à enroulement serré (28) est un ressort à enroulement serré (28) destiné à un guide-fil de manœuvre d'incurvation, lequel guide un fil (70) réalisant une manœuvre d'incurvation d'une portion incurvée d'endoscope (20).

8. Procédé de déformation plastique d'un ressort à enroulement serré (28) selon la revendication 7, dans lequel
le ressort à enroulement serré (28) est un ressort à enroulement serré (28) dans lequel un fil destiné à une manœuvre d'incurvation d'une portion incurvée de la portion d'introduction d'endoscope est introduit.
